# EUROPEAN PATENT APPLICATION

(11) **EP 2 264 425 A1**
(43) Date of publication of application: **22.12.2010**
(21) Application number: 09007953.4
(22) Date of filing: 18.06.2009
(51) Int. Cl.: G01N 1/32

(54) **Method and arrangement for polishing an implant sample**

(71) Applicant: Nobel Biocare Services AG, 8058 Zürich-Flughafen (CH)
(72) Inventor: Fäldt, Jenny, 431 34 Mölndal (SE); Grüner, Daniel, 106 91 Stockholm (SE); Shen, Zhijan, 106 91 Stockholm (SE)
(74) Representative: Byström, Kurt Linus

(57) **Abstract**

A method and arrangement is disclosed for polishing a surface of a sample, which comprises bone tissue (3), an implant portion (4), and an interface portion (4a) between the bone tissue (3) and the implant portion (4). The method comprises arranging between an Ar ion bean source (5) and the sample (2) a shield (6) having an edge surface (7) such that a first portion (2a) of the sample containing said surface to be polished is unshielded by the shield (6) at a first side of the edge surface (7) and a second portion of the sample (2b) is shielded by the shield (6) at a second side of the edge surface. The Ar ion beam source (5) is directed towards the edge surface (7). Then, the first portion (2a) of the sample (2) is exposed to Ar ions from the Ar ion beam source (5).

## Description

### Field of the Invention

The present invention relates to a method for polishing a surface of a sample containing materials of different properties, and in particular a sample comprising bone tissue, an implant portion, and an interface portion between the bone tissue and the implant portion. The invention also relates to an arrangement for polishing such a sample.

### Background of the Invention

Rapid and sustainable stability and functionality of bone anchored implants, such as dental implants, as well as optimal soft tissue healing may be achieved by improved surface structures of the implant. The surface of, e.g., dental implants has developed from a machined surface to a structured/rougher surface with better healing properties. One example of the latter is the TiUnite® surface provided by Nobel Biocare on dental implants, which is a more porous surface than a machined surface and that facilitates the integration of the implant compared to an implant with a machined surface by reducing bone resorption. To further guide and optimize the healing process, the implant surface can be coated with active substances. Such coatings can reduce inflammatory reactions leading to bone resorption, stimulate bone formation for faster osseointegration, or treat infections locally. A crucial prerequisite to enhance performance of implants is the quantitative analysis of the osseointegration and healing processes at the implant/tissue interface and their relation to microstructure and local chemistry.

Osseointegration is commonly characterized by light microscopy or scanning electron microscopy (SEM) observation of the surface of the implant, or a mechanically polished cross section thereof, where magnifications well below 1000 are used. These observations allow for determining the bone coverage of the whole implant (i.e., the fraction of the implant surface or interface area covered with bone). An issue with mechanical polishing is that artifacts arising from mechanical scratching of the interface between the implant and bone tissue limit the resolution at which the polished surface may be studied. Sometimes the sample includes material that is sensitive to mechanical polishing, such as bone tissue. The mechanical polishing may damage the bone tissue at the interface between bone tissue and implant surface, e.g. when the bone tissue is softer than the material of the surface of the implant.

Hence, an improved method and arrangement of facilitating polishing of an interface of a implant sample consisting of components with very different physical properties would be advantageous, which allows in particular for improved precision or accuracy, increased flexibility, cost-effectiveness, less prune to cause artifacts, and/or for improved quantitative analysis of the interaction of implant surface and bone tissue.

### Summary of the Invention

Accordingly, embodiments of the present invention preferably seek to mitigate, alleviate or eliminate one or more deficiencies, disadvantages or issues in the art, such as the above-identified, singly or in any combination by providing methods, and arrangement according to the appended patent claims.

According to a first aspect, a method is provided for polishing a surface of a sample comprising bone tissue, an implant portion, and an interface portion between the bone tissue and the implant portion. The method comprises arranging between an Ar ion bean source and the sample a shield having an edge surface such that a first portion of the sample, which contains the surface to be polished, is unshielded by the shield at a first side of the edge surface and a second portion of the sample is shielded by the shield at a second side of the edge surface. The Ar ion beam source is directed towards the edge surface. Furthermore, the first portion of the sample is exposed to Ar ions from the Ar ion beam source.

The method may comprise pre-polishing the surface to be polished to a first level of surface finishing followed by further polishing of the surface to a second level of surface finishing using the Ar ion beam source. The first level of surface finishing may be rougher than the second level of surface finishing. The pre-polishing may comprise mechanical pre-polishing and/or chemical pre-polishing.

The method may also comprise pre-polishing another surface of the sample such that the surface to be polished and the other surface are provided at a substantially 90 degrees angle.

The method may comprise arranging the surface to be polished offset from the edge surface in the lateral direction as viewed from the Ar ion beam source, such as approximately 25-100 µm, preferably approximately 50-75 µm, from the edge surface.

The method may comprise partially embedding the sample in resin prior to providing the shield over the sample.

The method may comprise directing the Ar ion beam source substantially parallel to the edge surface.

The method may comprise attaching the sample to a support.

The method may comprise exposing the first portion of the sample to the Ar ions until at least a portion of the interface portion is polished. The exposure time may be a predetermined period of time, preferably 10 to 30 hours.

The method may comprise polishing the first portion over a width of at least 200 µm, preferably at least 500 µm, as measured along the edge surface The method may comprise polishing the first portion over a depth of at least 300 µm, preferably at least 500 µm, as measured from a top end to a bottom end of the polished surface.

According to a second aspect, an arrangement is provided for polishing a surface of a sample, which comprises bone tissue, an implant portion, and an interface portion between the bone tissue and the implant portion. The arrangement comprises an Ar ion beam source, a sample having a surface to be polished, which contains the bone tissue, the implant portion, and the interface portion, and a shield having an edge surface and being sized to expose a first portion of the sample, which comprises the surface to be polished, to Ar ions from the Ar ion beam source and shield a second portion of the sample from Ar ions from the Ar ion beam source.

The surface to be polished and the edge surface may be arranged offset in the lateral direction as viewed from the Ar in beam source. In some embodiments, the surface to be polished and the edge surface are arranged substantially parallel. In some embodiments, the surface to be polished and the edge surface are arranged offset and substantially parallel.

The shield may be made of metal, such as nickel or molybdenum.

The sample may be at least partially embedded in resin. In some embodiments, the sample is attached to a support. In some embodiments, the sample is embedded in resin and attached to a support.

According to a third aspect, a sample contains bone tissue, an implant portion, and an interface portion between the bone tissue and the implant portion. The sample has a surface polished using any of the embodiments of the method described herein.

Embodiments of the invention provides for studying a polished interface between bone tissue and an implant in a SEM, up to 1 mm wide of the interface, containing some 100 pores in the case of implants contain a porous surface with pores in the low micrometer range. This information allows for a quantitative analysis of the pore filling with bone, thereby extending the bone coverage measurements typically performed on mechanically polished cross-sectioned implants to the sub-micrometer regime. Implant/bone interfaces may conveniently be analyzed using BSE images where Ti, bone, coating and resin/soft tissue can easily be distinguished due to atomic number contrast and the sharp boundaries between the different materials. The characterization of the local chemistry is possible by EDS spectrometry.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a flowchart of an embodiment of a method for polishing a surface of an implant sample;
Fig. 2 is a schematic view of an arrangement for polishing a surface of an implant sample;
Fig. 3 is an implant sample polished according to an embodiment of the method for polishing a surface of an implant sample; and
Fig. 4 is a backscattered electron image of an implant sample polished according to an embodiment.

### Detailed Description of Embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

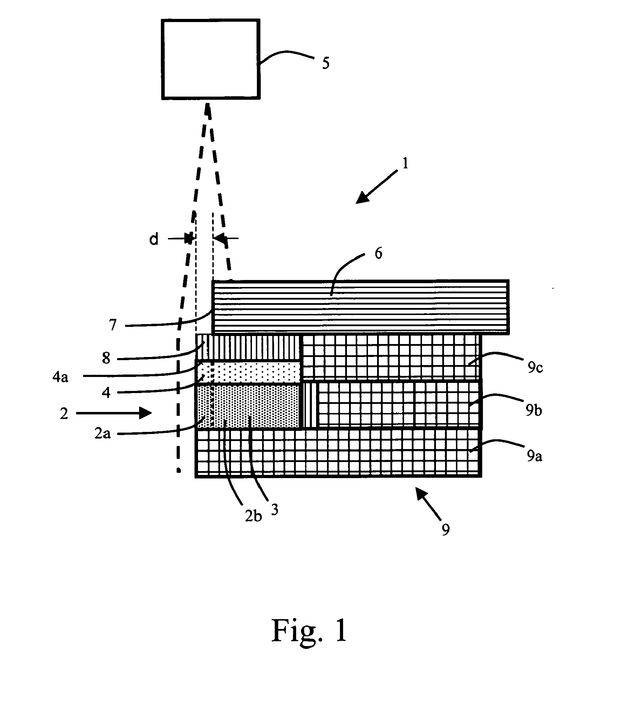

Fig. 1 illustrates an embodiment of an arrangement 1 for polishing a surface of á sample 2 comprising bone tissue 3, an implant portion 4, and an interface portion 4a between the bone tissue 3 and the implant portion 4. The arrangement further comprises an Ar (argon) ion beam source 5. The sample has a surface to be polished. The surface to be polished contains at least a portion of the bone tissue 3, at least a portion of the implant portion 4, and the interface portion 4a. Furthermore, the arrangement comprises a shield 6 for covering at least a portion of the sample 2, and thus at least partially protect from exposure to ions of an Ar ion beam from the Ar ion beam source 5. The shield 6 may be a shield plate. The shield 6 has an edge surface 7. The edge surface 7 may be a lateral edge of a plate having a non-zero thickness. Also, the shield 6 is sized and/or can be arranged to expose, i.e. not cover, a first portion 2a of the sample 2, which comprises the surface to be polished, to the Ar ion beam from the Ar ion beam source 5. Furthermore, the shield 6 at least partially protects a second portion 2b of the sample from ions of the Ar ion beam. Hence, the first portion 2a, which is arranged on a first side of the edge surface 7, may be completely exposed to ions from the Ar ion beam source 5, whereas the shield 6 may shield the second portion 2b of the sample 2, which is arranged on a second side of the edge surface 7. The shield 6 is arranged between the Ar ion beam source 5 and the sample 2. Hence, the first portion 2a of the sample 2 protrudes from the shield 6 as viewed from the Ar ion beam source 5. The thickness of the plate may be in the range of 1.0-2.5 mm. The specific dimensions of the shield depend on the area of the sample 2 that should be shielded The interface portion 4a may comprise a surface of the implant portion 4, at which bone tissue 3 has or ought to be osseointegrated. There may also be a gap between the bone tissue 3 and the surface of the implant portion 4. In some embodiments, the surface of the implant portion 4 is a porous surface containing pores with size in the meso (2-50nm) or macro (larger than 50 nm) region. One example of such a surface is the above TiUnite® surface, which has a pore diameter in the range of 0.1-10µm, provided by Nobel Biocare and being subject of International patent publication No. WO00/72776, which is incorporated herein in its entirety by reference for any purpose.

In some embodiments, the surface to be polished and the edge surface 7 are arranged offset. The offset may be a lateral offset as viewed from the Ar ion beam source 5. The offset may depend on the depth d of the portion of the sample that should be polished. The desired depth may e.g. depend on the roughness of the surface to be polished. In some embodiments the depth is in the range of 25-100 µm, preferably in the range of 50-75 µm. The latter range may e.g. by relevant when the surface to be polished has been pre-polished, such as mechanically or chemically, as will be described further below.

In some embodiments, the edge surface 7 and the surface to be polished are arranged substantially parallel, such as by aligning the edge surface 7 with the centre axis of the Ar ion beam. This allows for polishing with a uniform depth d of the surface to be polished. This also allows for providing the Ar ions substantially parallel to the edge surface 7, whereby the polished surface after polishing will be substantially parallel to the edge surface 7.

As discussed above, the shield 6 stops at least a portion of the Ar ion beam. The portion stopped may e.g. be in the range of 40-60%, such as 50%. In some embodiments, the shield 6 is made of metal, such as nickel or molybdenum.

In the embodiment of Fig. 1, the sample 2 is partially embedded in resin 8. In other embodiments, the sample is completely embedded in the resin 8. The resin 8 may e.g. be an epoxy resin, such as G-2, available via Gatan, USA, or a light-curing one-component resin, such as Technovit 7200 VLC, available via Heraeus Kulzer, Germany. Embedding the sample 2 at least partially in the resin 8 allows for providing a substantially flat surface, upon which the shield 6 may be positioned. This, in turn, allows for a more stable arrangement 1, whereby improved polishing can be achieved since artefacts are avoided if there are no gaps between the sample 2 and the shield plate 6. This may be particularly useful if the implant portion 5 e.g. has en irregular or uneven outer surface that is difficult to support.

In some embodiments, the sample 2 is attached or rests on a support 9, potentially at least partially via the resin 8 if the sample 2 is embedded within it. In some embodiments, the support 9 comprises a plurality of sub-supports 9a, 9b, 9c. In the embodiment of Fig. 1, three sub-supports 9a, 9b, 9b are provided. The sample 2 is positioned on a first sub-support 9a, which also supports a second sub-support 9b. A third sub-support 9c is supported by the second sub-support 9b. A top surface of the third sub-support 9c may substantially level with a top surface of the sample 2 or the resin 8 if the former is embedded in the latter. Hence, the shield 6 may be supported by the third sub-support 9c and the sample 2 and/or resin 8. This allows for arranging the edge surface 7 substantially parallel with the surface to be polished when the edge surface 7 is substantially perpendicular with the surface of the shield 6 that rests on the third sub-support 9c. The support 9 is particularly useful for a small specimen of a fresh implant. In the case of a bone/implant sample, a block cut out from the sample/resin may be self-supporting and have plan-parallel supporting planes.

The support 9 and/or sub-supports 9a-9c may comprise Si disks or glass disks cut from microscope slides or cover slips. Alternatively, any other type of material with a plan-parallel surface can be used for he support 9.

In other embodiments, the sub-supports 9a-9c are provided as an integral unit. Alternatively, more than three sub-supports 9a-9c are provided, e.g. depending on the thickness of the sample 2 and/or resin 8.

The Ar ion beam source 5 may e.g. be comprised in an Ar ion polishing apparatus. Such an apparatus may e.g. be the SM-09010 Cross-sectional polisher available from JEOL, Japan. The accelerating voltage of the argon ions is adjustable in the range 2-6 kV with beam currents between 20-150 µA. Ar ion polishing apparatus may comprise a sample chamber, a control unit. The Ar ion beam source may be an Ar ion gun of the Ar ion polishing apparatus.

In some embodiments, the bone tissue 3 is arranged further away from the Ar ion beam source 5 than the implant portion 4 of the sample 2. Consequently, the implant portion 4 is arranged closer to the Ar ion source 5 than the bone tissue 3. This has, i.a., the advantage that the damaging of the Ar ion beam sensitive material, such as the bone tissue 3 and/or resin 8, by the Ar ion beam can be reduced. Such damaging can sometimes be caused when the beam sensitive material is polished before the implant portion 4, such as when the implant portion 4 is made of metal, e.g. titanium, or ceramic, e.g. zirconia or alumina or their composites with hydroapatite. Hence, artifacts can be avoided. Polishing the implant portion 4 before bone tissue minimizes both beam exposition of the beam-sensitive materials and deposition of the implant portion 4 material in gaps between the bone tissue 3 and the implant portion 4. The resin 8 may still be polished before the implant portion 4.

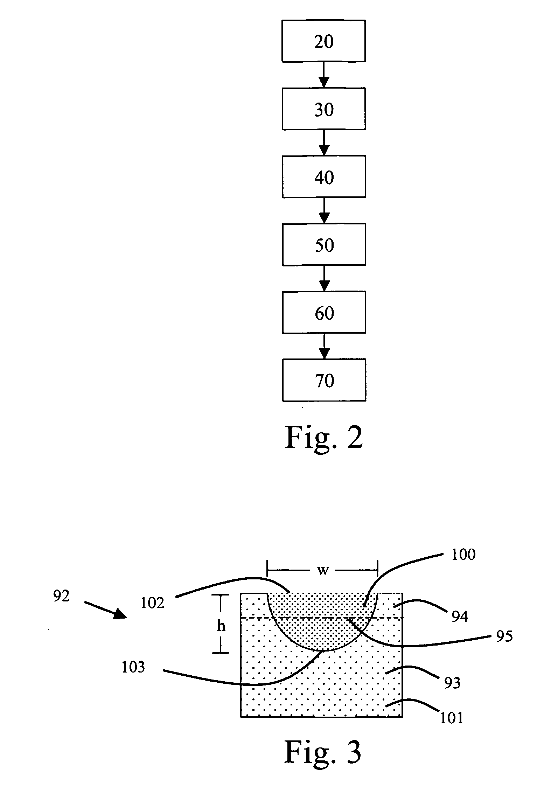

Fig. 2 illustrates a flow chart of an embodiment of a method for polishing a surface of the sample 2. The sample 2 is extracted after an in vivo test. Hence, the method may be an in vitro method or an ex vivo method. The sample 2 comprises the bone tissue 3, the implant portion 4, and the interface portion 4a between the bone tissue 3 and the implant portion 4.

At 20 the sample 2 is at least partially embedded in the resin 8 prior to providing the shield 6 over the sample 2.

At 30 at least one surface of the sample is pre-polished. The pre-polished surface may be the surface to be polished by Ar ions. In other embodiments, additionally or alternatively, another surface of the sample 2 is pre-polished. Hence, the surface to be polished and the other surface may be provided at a substantially 90 degrees angle. This allows for supporting the sample 2 on a surface that is perpendicular to the exposure direction of the Ar ion beam. Also, this allows for arranging the edge surface 7 and/or the surface of the sample 2 that is to be polished parallel to the incident beam from the Ar ion source 5.

In some embodiments, the pre-polishing comprises mechanical pre-polishing. The mechanical pre-polishing may e.g. comprise grinding with, optionally, using a lubricant. Grinding may e.g. be carried out by using a 400-grit SiC paper, or using 1-100 micrometer sized abrasives composed of diamond, cubic boron nitride, silicon carbide, silica, alumina. The lubricant may e.g. comprise water, ethanol, ethylene glycol, or mixtures thereof.

The surface to be polished by Ar ions may be pre-polished to a first level of surface finishing followed by further polishing to a second level of surface finishing using the Ar ion beam source. The first level of surface finishing is rougher than the second level of surface finishing. Pre-polishing of the surface to be polished by Ar ions allows for less need for removal of material from the sample, hence reducing the polishing time and the redeposition of the polished-away materials. Consequently, the distance d at which the sample 2 protrudes from the shield 2 may be reduced.

At 40 the sample 2, potentially embedded in the resin 8, is attached to the support 9. The sample 2 may be attached using an adhesive, such as carbon paint, for example Conductive Carbon Cement available from Plano, Germany. The arrangement 1, including support 9 if used, may be attached to a sample holder of the Ar ion polishing apparatus.

At 50, the sample 2 is arranged between the Ar ion beam source 5 and the shield 6, which has the edge surface 7. The sample 2 is arranged such that the first portion 2a of the sample 2, which contains the surface to be polished by the Ar ions, is unshielded by the shield 6 at a first side of the edge surface 7. Furthermore, the sample 2 is arranged such that the second portion 2b of the sample 2 is shielded by the shield 7 at a second side of the edge surface. Hence, the second portion 2b protrudes from the edges surface 7 of the shield 6. Consequently, a lateral distance is provided between the surface to be polished by the Ar ions, such as the pre-polished surface, and the edge surface 7 before polishing commences.

In some embodiments, the surface to be polished by Ar ions is arranged offset from the edge surface 7 in the lateral direction as viewed from the Ar ion beam source. The offset corresponds to the distance d discussed above. The offset is in some embodiments approximately 25-100 µm, preferably approximately 50-75 µm.

At 60, the Ar ion beam source 5 is directed towards the edge surface 7 and thus towards the first portion 2a of the sample 2. The Ar ion beam source may be directed such that the Ar ion beam, such as a centre axis of the beam cone from the Ar ion beam source, is positioned substantially axially and co-aligned with the edge surface 7. Hence, the Ar ion beam is in such a set-up substantially parallel and co-axial with the edge surface. This provides for maximum exposure of the first portion 2a.

At 70, the first portion 2a of the sample 2 is exposed to at least a portion of the Ar ions from the Ar ion beam source 5. The first portion 2a of the sample 2 may be exposed to the portion of the Ar ions until a region of interest has been polished. The region of interest may e.g. comprise at least portions of the bone tissue 3, the interface portion 5, and the implant portion 4. Ar ion polishing may be performed a predetermined period of time, preferably 10 to 30 hours, depending on the thickness and/or hardness of the materials to be polished.

The sample 2 may be exposed at the first portion 2a to the Ar ion beam over a width w (see Fig. 3) of at least 200 µm, preferably at least 500 µm, as measured along the edge surface. The width may be in the range of 200-1000 µm, preferably in the range of 500-1000 µm. Since the arrangement 1 may be rocking during the polishing, the polished width may be larger than the diameter of the Ar ion beam at the edge surface 7. Additionally or alternatively, the sample 2 may be exposed at the first portion 2a to the Ar ion beam to a depth of at least 300 µm, preferably at least 500 µm, of the sample surface as measured from a surface of the shield, which faces towards the sample 2 and is perpendicular to the edge surface 7 and parallel to an exposure direction of the Ar ion beam. Hence, the first portion 2a is polished over a width of at least 200 µm, preferably at least 500 µm, as measured along the edge surface 7, and a depth of at least 300 µm, preferably at least 500 µm, as measured from a top end of the polished surface.

At least part of the first portion 2a of the sample 2 that protrudes from the shield 6 is slowly milled by the Ar ion beam, leaving behind a polished surface at the position of the edge of the shield 6. At this position, the incidence of Ar ions is parallel to the surface, e.g. if the centre of the Ar ion beam cone from the Ar ion beam source 5 is arranged substantially parallel with the edge surface 7.

Various embodiments of the method for polishing the surface of the sample 2 include only some or all of the steps discussed above. For example, all of the steps 20, 30, and 4 may be omitted or included independently of each other. However, each of these steps add e.g. to the stability of the arrangement, allowing for polishing with less artifacts, which provides for the positive effects discussed above.

Fig. 3 is a side view of a surface of a sample 92 containing bone tissue 93, an implant portion 94, and an interface portion 95 between the bone tissue 93 and the implant portion 94. The sample 92 has been polished using the arrangement according to any of the embodiments of Fig. 1 described above, and any of the methods described above with reference to fig. 2. Hence, the sample 92 has a first surface 100 polished using the Ar ion beam source 5. Furthermore, the sample 92 has a second surface 101 at least partially surrounding the first surface 100. The first surface 100 is smoother than the second surface 101. The second surface 101 may be pre-polished, as described above. The first surface has a width w at a top end 102 thereof. The width w is in some embodiments in the range of 200-1000 µm, such as 300-700 µm. The top end 102 faced towards the Ar ion beam source 5 during polishing. The first surface 100 has a height h as measured from the top end 102 to a bottom end 103 thereof, i.e. as measured to the lowermost end of surface 100 as seen from the top end 102. The height h is measured parallel to the exposure direction of the Ar ion beam, such as perpendicularly to a top surface of the sample 92 or the resin, if the sample is embedded in the latter. The height h is in some embodiments at least 300 µm, preferably at least 500 µm. The depth is dependent on the exposure time for the Ar ion beam and may be selected such as to polish the region of interest of the sample 92.

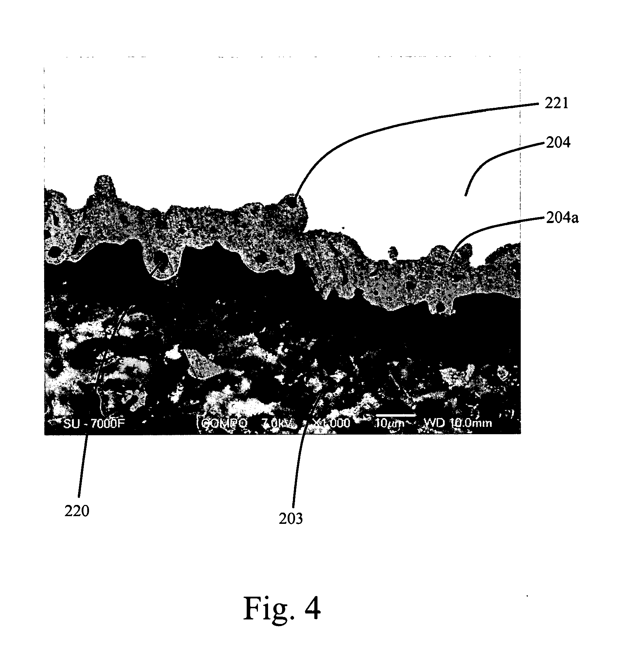

Fig. 4 is a backscattered electron image of a sample polished according to an embodiment. The sample 202 comprises bone tissue 203, an implant portion 204, and an interface portion 204a between the bone tissue 203 and the implant portion 204. In this interface, the interface portion 204a comprises a surface of the implant portion 204 having pores 220, 221. The interface portion 204 is visible as lighter grayish area. A pore filled with bone substance is clearly visible as a darker grayish area within the lighter graying area, e.g. as indicated by reference numeral 220, and clearly distinguishable from a pore not filled with bone substance visible as a black area, e.g. as indicated by reference numeral 221. This clearly shows that neither the empty pores 221 nor the pores 220 filled with bone tissue are contaminated by particles from the polishing media or the polishing process. Each material is clearly distinguishable.

### Example 1

In a first example, from each implant an approx. 3 mm wide piece was cut using a diamond cutting blade. The cylindrical pieces were then grinded to half cylinders, turned and grinded to one quarter of a cylinder with the polished faces forming an angle of approx. 90 degrees. For the grinding operations, 400-grit SiC paper with water as lubricant was used. After grinding, the samples were thoroughly cleaned with water and acetone and glued onto Si disks. The samples were then embedded in epoxy resin (G-2, Gatan, USA) which was cured within 1 h at 100 °C. Prior to the cross-section polishing, the embedded samples were polished mechanically (400 grit SiC paper, water as lubricant) to ensure that the sample surface was parallel to the incident beam. Finally, Ar ion milling was performed as disclosed above at 4 kV / 70 µA for around 20h.

The Ar ion polished surfaces of example 1 were studied using a JSM-7000F field emission scanning electron microscope (JEOL, Japan) equipped with an energy dispersive X-ray (EDX) spectrometer (Oxford Instruments, UK). Accelerating voltages in the range 5-10 kV were used for secondary electron (SE) and backscattered electron (BSE) imaging and recording EDX spectra. In a BSE image the Ti/coating interface was clearly visible as a sharp line. A small gap between the resin (dark) and the coating could be seen. The coating comprised unevenly distributed pores with diameters in the range of 1-5 µm. Secondary electron images at higher magnification revealed the presence of an approx. 1 µm thick layer at the Ti/coating interface which comprised a high density of small pores (20-200 nm). Larger pores were observed more than 1 µm away from the interface.

### Example 2

In a second example, samples were prepared to study the implant/bone interface. The bone in this case was jaw bone of a mini-pig. The implants were made of Ti with a coating comprising Ti, O, and P. The bone was cut longitudinally in the mesio-distal direction of the jaw and through the middle of the implants using an Exakt cutting unit (Exakt, Germany) equipped with a diamond-coated band saw. The two resulting halves of the original specimen were embedded in resin following complete dehydration in ascending grades of ethanol in a light-curing one-component resin (Technovit 7200 VLC, Heraeus Kulzer, Germany).

From one half of the original specimen, prismatic blocks with approximate dimensions 1 × 5 × 5 mm³ containing a region close to the tip of the implant were cut using a low-speed saw (Isomet, Buehler, USA) and diamond wafering blades. These blocks were mechanically polished as described with regard to example 1 above and then fixed on the sample holder of the cross-section polishing apparatus using carbon paint (Conductive Carbon Cement, Plano, Germany). Argon milling was performed at 3.5 kV / 50 µA for 22-24 h.

BSE images of a polished cross section of the implant bone interface in example 2 were studied. Ti, coating, bone and resin/soft tissue could clearly be distinguished due to atomic number contrast. Therefore, bone tissue filled and unfilled pores inside the coating were easily recognized. At higher magnification it could be seen that pores close to the bone layer were almost completely filled whereas a large pore close to the Ti only partially was filled with bone.

BSE images of a polished cross section of the implant bone interface of example 2 were studied. Ti, coating, bone and resin/soft tissue could clearly be distinguished due to atomic number contrast. A filled pore and a partially filled pore could be seen. Bone contacted the oxide layer not only on the surface but also inside pores.

Embodiments of the invention provide for improved polishing, wherein artefacts can be reduced compared to mechanical polishing. This is especially useful for analysing samples containing harder and softer materials, wherein the region of interest is in the interface between the harder and softer materials. Such harder material may be an implant for implantation into the softer material, such as bone tissue. Embodiments of the invention are particularly useful for analysing large areas of porous surfaces from few hundreds to above ten thousand of square micrometers, wherein the pores have diameters are in the meso (2-50nm) or macro (larger than 50 nm) range, such as submicron up to tens of micrometers, e.g. 0,1-20 µm. Other techniques, such as focused ion beam (FIB), are useless for this purpose due to its small polishing capabilities. For example, using FIB areas of up to only 20 µm can be polished. Hence, analysing a sample containing a porous surface would only allow including one or a few pores. For larger pores, the area to be polished would have to be centred around a pore to fully capture it. Since the arrangement and method according to embodiments of the invention provide for polishing of larger areas, more pores, often fewer tens or more than one hundred, can be captures on the polished areas without suffering of the drawbacks of FIB polishing. Hence, the invention provides for improved quantitative analysis of the interaction of implant surface and bone tissue. At the same time, artefacts, and damage of the region of interest as well as contamination with particles from the polishing media, can be substantially reduced, if not completely avoided, which is an issue of concern with mechanical polishing.

The present invention has been described above with reference to specific embodiments. However, other embodiments than the above described are equally possible within the scope of the invention. Different method steps than those described above may be provided within the scope of the invention. The different features and steps of the invention may be combined in other combinations than those described. The scope of the invention is only limited by the appended patent claims.

## Claims

1. A method for polishing a surface of a sample (2) comprising bone tissue (3), an implant portion (4), and an interface portion (4a) between the bone tissue (3) and the implant portion (4), the method comprising
arranging between an Ar ion bean source (5) and the sample (2) a shield (6) having an edge surface (7) such that a first portion (2a) of the sample containing said surface to be polished is unshielded by the shield (6) at a first side of the edge surface (7) and a second portion of the sample (2b) is shielded by the shield (6) at a second side of the edge surface,
directing the Ar ion beam source (5) towards the edge surface (7); and
exposing the first portion (2a) of the sample (2) to Ar ions from the Ar ion beam source (5).

2. The method according to claim 1, comprising pre-polishing the surface to be polished to a first level of surface finishing followed by further polishing of said surface to a second level of surface finishing using said Ar ion beam source (5), wherein the first level of surface finishing is rougher than the second level of surface finishing.

3. The method according to claim 2, wherein the pre-polishing comprises mechanical pre-polishing.

4. The method according to any of the previous claims, comprising pre-polishing another surface of the sample such that the surface to be polished and the other surface are provided at a substantially 90 degrees angle.

5. The method according to any of the previous claims, comprising arranging the surface to be polished offset from the edge surface (7) in the lateral direction as viewed from the Ar ion beam source (5), such as approximately 25-100 µm, preferably approximately 50-75 µm, from the edge surface (7).

6. The method according to any of the previous claims, comprising partially embedding the sample (2) in resin (8) prior to providing said shield (6) over the sample (2).

7. The method according to any of the previous claims, comprising directing the Ar ion beam source (5) substantially parallel to the edge surface.

8. The method according to any of the previous claims, comprising attaching the sample (2) to a support (9).

9. The method according to any of the previous claims, comprising exposing the first portion (2a) of the sample (2) to the Ar ions until at least a portion of the interface portion is polished, such as for predetermined period of time, preferably 10 to 30 hours.

10. The method according to any of the previous claims, comprising polishing the first portion (2a) over a width (w) of at least 200 µm, preferably at least 500 µm, as measured along the edge surface, and a depth (d) of at least 300 µm, preferably at least 500 µm, as measured from a top end (102) to a bottom end (103) of the polished surface.

11. An arrangement for polishing a surface of a sample (2) comprising bone tissue (3), an implant portion (4), and an interface portion (4a) between the bone tissue (3) and the implant portion (4a), comprising an Ar ion beam source (5);
a sample (2) having a surface to be polished, the surface containing the bone tissue (3), the implant portion (4), and the interface portion (4a); and
a shield (6) having an edge surface (7) and being sized to expose a first portion (2a) of the sample (2), which comprises the surface to be polished, to Ar ions from the Ar ion beam source (5) and shield a second portion (2b) of the sample (2) from Ar ions from the Ar ion beam source (5).

12. The arrangement according to claim 11, wherein the surface to be polished and the edge surface (7) are arranged offset in the lateral direction as viewed from the Ar in beam source (5), and preferably substantially parallel.

13. The arrangement according to claim 11 or 12, wherein the shield (6) is made of metal, such as nickel or molybdenum.

14. The arrangement according to claim any of claims 11-13, wherein the sample (2) is at least partially embedded in resin (8), and preferably attached to a support (9).

15. A sample (2) containing bone tissue (3), an implant portion (4), and an interface portion (4a) between the bone tissue (3) and the implant portion (4a), the sample (2) having a surface polished using the method according to any of the claims 1-10.
